# EUROPEAN PATENT SPECIFICATION

(11) **EP 3 121 238 B1**
(45) Date of publication and mention of the grant of the patent: **20.11.2019**
(21) Application number: 15765588.7
(22) Date of filing: 18.03.2015
(51) Int. Cl.: C09K 3/00, A61L 9/01, A61L 9/14, D06M 11/42, D06M 11/44, E04F 13/07, E04F 15/02

(54) **ALLERGEN-REDUCING COMPOSITION, SPRAY AGENT AND SURFACE TREATING AGENT CONTAINING SAID COMPOSITION AND ALLERGEN-REDUCING METHOD WITH SAID COMPOSITION**
ALLERGENREDUZIERENDE ZUSAMMENSETZUNG, SPRÜHMITTEL UND OBERFLÄCHENBEHANDLUNGSMITTEL MIT BESAGTER ZUSAMMENSETZUNG UND ALLERGENREDUZIERENDES VERFAHREN MIT BESAGTER ZUSAMMENSETZUNG
COMPOSITION ANTI-ALLERGÉNIQUE, AGENT DE PULVÉRISATION ET AGENT DE TRAITEMENT DE SURFACE LA CONTENANT ET PROCÉDÉ DE RÉDUCTION DES ALLERGÈNES AVEC CETTE COMPOSITION

(30) Priority: 20.03.2014 JP 2014057433; 30.10.2014 JP 2014221120
(43) Date of publication of application: 25.01.2017
(73) Proprietor: SC Environmental Science Co., Ltd., Chuo-ku Osaka-shi Osaka 541-0045 (JP)
(72) Inventor: INUI, Keiichiro, Nishinomiya-shi Hyogo 663-8242 (JP)
(74) Representative: Reitstötter Kinzebach
(86) International application number: PCT/JP2015/058021
(87) International publication number: WO 2015/141713

(56) References cited:
- WO-A1-03/075664
- CN-A- 100 998 942
- CN-A- 103 891 776
- JP-A- 2006 239 393
- JP-A- 2011 056 471
- JP-A- 2011 256 283
- JP-A- 2012 144 635
- JP-A- 2012 144 635
- JP-A- 2013 181 102
- US-A1- 2002 040 055
- US-A1- 2010 069 338
- CARRIE I MORGAN ET AL: "Zinc supplementation alters airway inflammation and airway hyperresponsiveness to a common allergen", JOURNAL OF INFLAMMATION, BIOMED CENTRAL, LONDON, GB, vol. 8, no. 1, 7 December 2011 (2011-12-07), page 36, XP021093771, ISSN: 1476-9255, DOI: 10.1186/1476-9255-8-36

## Description

### TECHNICAL FIELD

The present invention relates to an allergen reducing composition and an allergen reducing method for reducing allergens such as mites and pollen; or an allergen reducing composition and an allergen reducing method for imparting a function of reducing allergens to unwoven fabric, textile or a textile product, or an architectural interior material.

### BACKGROUND ART

A lot of people are afflicted with allergic diseases such as asthma, atopic dermatitis, and allergic rhinitis, and particularly in recent years, the allergic diseases have a tendency to increase. A variety of allergens which are present in the environment cause these allergic diseases. Among the allergens, mites, pet hairs, pollen, and mold living indoors have been well known as inhalant allergens. In particular, Epidermoptidae which is a kind of house dust mites inhabiting the inside of a house has posed the serious problem as a causal agent of the allergens. Indoor textile products such as a tatami mat, a carpet, bedding, and a curtain or outdoor textile products such as seat fabric of a mobile vehicle such as a train and an automobile are hotbeds for growing the Epidermoptidae.

Among the Epidermoptidae, Dermatophagoides farinae and Dermatophagoides pteronyssinus are representative species, and dead bodies and feces of these mites become strong allergenic substances. Moreover, in addition to pollen of cedars (Cryptomeria japonica) scattering in early spring, pollen of a variety of plants also acts as allergens and in particular, causes the sideration of the allergic rhinitis. As the scattering pollen, not only the cedar pollen scattering in early spring, but also there are many kinds of pollen of plants such as hinoki cypresses, mugworts, ragweeds, and orchard grass, and some kinds of pollen are scattering around through the year. It is considered that at any time of a year, a risk of inducing the allergy caused by the pollen is posed.

In order to remove these allergens such as the mites and the pollen, there employed is a method in which the air is filtered by using an air conditioner or an air purifier. However, only the allergens scattering in the air can be removed, resulting in the accumulation of the allergenic substances on the filter, and upon replacing the filter, a risk of scattering the allergenic substances again is posed. In addition, a mask is used to prevent the pollen of the cedar or the like from being inhaled. However, since the allergens of the pollen adhering to the mask do not vanish, a risk of scattering the allergenic substances again and being thereby inhaled is posed. Using an electric vacuum cleaner is effective as the method of removing the allergens. However, a large amount of allergens included in dust sucked thereinto is merely stored in a dust bag, and a risk of scattering the allergenic substances again upon the disposal of the dust bag is considered.

As a chemical agent to reduce or remove allergenic properties of the allergenic substances, tannic acid has been known since long ago, and a method of applying a polyphenol compound such as the tannic acid has been proposed. For example, there disclosed are a method (Patent Literature 1) in which the tannic acid is used as a deactivator for the allergens, and a method (Patent Literature 2) in which compounds of tea extract, hydroxyapatite, epicatechin, epigallocatechin, epicatechin gallate, epigallocatechin gallate, gallic acid, and ester compounds of gallic acid and 1-4C alcohol are used as deactivators for the allergens.

As an allergen reducing composition using a zinc compound, an inorganic salt of zinc such as zinc oxide, zinc chloride, zinc sulfate, zinc citrate, zinc gluconate, zinc lactate, zinc silicate and zinc nitrate; and a water-soluble zinc compound such as zinc acetate has been proposed as a reducing component, a removing agent, a neutralizing composition for the allergens or a deactivator for the allergens (Patent Literatures 3 to 9 and Non-Patent Literature 1). In addition, an allergen reducing composition using a rare earth salt has been proposed (Patent Literatures 10 and 11). Further, processing an antiallergenic composition containing a hydroxide of a metal such as rare earths, cobalt, nickel, copper, and zinc and anatase-type titanium dioxide into a coating agent, a resin product, a wallpaper, a nonwoven fabric, a fiber, or a fiber product has been described (Patent Literature 12).

### CITATION LIST

### PATENT LITERATURE

Patent Literature 1: Japanese Patent Application Laid-Open Publication No.S61-44821
Patent Literature 2: Japanese Patent Application Laid-Open Publication No. H6-279273
Patent Literature 3: Japanese Patent Application Laid-Open Publication No. 2006-239393
Patent Literature 4: Japanese Patent Application Laid-Open Publication No. 2007-39620
Patent Literature 5: Japanese Patent Application Laid-Open Publication No. 2003-96670
Patent Literature 6: Japanese Patent Application Laid-Open Publication No. 2004-189606
Patent Literature 7: Japanese Unexamined Patent Application (Translation of PCT Application) 2004-510841
Patent Literature 8: US Patent Application Laid-Open Publication No.2010/0069338
Patent Literature 9: WO Patent Application Laid-Open Publication No. 03/075664
Patent Literature 10: Japanese Patent Application Laid-Open Publication No. 2001-322937
Patent Literature 11: US Patent Application Laid-Open Publication No. 2002/0040055
Patent Literature 12: Japanese Patent Application Laid-Open Publication No. 2012-144635

Non-Patent Literature 1: Morgan et al., Journal of Inflammation 2011, 8(36): 1-9

### SUMMARY OF THE INVENTION

### TECHNICAL PROBLEM

However, although the tannic acid as disclosed in Patent Literature 1 is an excellent agent because the tannic acid is a highly safe natural substance and has an excellent allergen reducing efficacy, the tannic acid has a brown color and further, the coloring proceeds over time, thereby leading to a problem in that applications are limited.

The zinc compounds do not have the problem related to the limited applications caused by the coloring as with the tannic acid. However, an effect of reducing the allergens of a water-insoluble salt such as the zinc oxide is not sufficient; and the water-soluble zinc compound such as the zinc chloride, the zinc sulfate, the zinc nitrate, and the zinc acetate is highly toxic and falls under the category of a deleterious substance, and when the water-soluble zinc compound is added to an emulsified dispersive substance such as resin emulsion, agglomeration occurs, thereby leading to a problem in that it is difficult to put the zinc compounds into practical use as the allergen reducing composition.

Efficacy of reducing the allergens of the allergen reducing composition using the rare earth salt disclosed in Patent Literature 10 is also not necessarily satisfactory.

Therefore, the aim of the present invention are to provide an allergen reducing composition which is capable of reducing allergens such as mites and cedar pollen and of imparting a function of reducing the allergens to unwoven fabric, textile, or a textile product and which causes less coloring of an allergen reduction target; a spray agent using the allergen reducing composition and a surface treatment agent using the allergen reducing composition; and an allergen reducing method.

### SOLUTION TO PROBLEM

In order to solve the above-described problems, the present inventors have conducted intensive researches. As a result, amazingly, the present inventors found that a composition containing compounds of both of a zinc salt or a copper salt and a rare earth salt exhibited a remarkably high synergistic effect of reducing allergens and this composition was capable of reducing the allergens such as mites and cedar pollen and completed the present invention.

Namely, an allergen reducing composition according to the present invention includes: (A) one or more kinds of compounds selected from the group consisting of a zinc salt of gluconic acid, citric acid, lactic acid or malic acid and copper gluconate; and (B) one or more kinds of compounds selected from the group consisting of rare earth salts. In the allergen reducing composition according to the present invention, it is preferable that the zinc salt is zinc gluconate. Further, in the allergen reducing composition according to the present invention, it is preferable that the rare earth salt is one or more kinds of compounds selected from the group consisting of a lanthanum salt, a cerium salt, a gadolinium salt, an erbium salt, and an ytterbium salt and it is more preferable that the rare earth salt is the lanthanum salt or the cerium salt.

In addition, in the allergen reducing composition according to the present invention, it is preferable that a mixing ratio of (A) the one or more kinds of compounds selected from the group consisting of the zinc salt of gluconic acid, citric acid, lactic acid or malic acid and copper gluconate and (B) the one or more kinds of compounds selected from the group consisting of the rare earth salts is 1 : 9 to 9 : 1 in a weight ratio.

In addition, it is preferable that the allergen reducing composition according to the present invention includes 0.5 to 70 wt.% (A) and (B) in total, the (A) being the one or more kinds of compounds selected from the group consisting of the zinc salt of gluconic acid, citric acid, lactic acid or malic acid and copper gluconate, the (B) being the one or more kinds of compounds selected from the group consisting of the rare earth salts.

In addition, a spray agent or a surface treatment agent according to the present invention contains: any of the above-mentioned allergen reducing compositions; and water or a water-soluble solvent.

In addition, an allergen reducing method according to the present invention is to conduct processing using any of the above-mentioned allergen reducing compositions in an environment where allergens are present. In the allergen reducing method according to the present invention, it is preferable that any of the above-mentioned allergen reducing compositions is caused to be present in an allergen reduction target space or on a surface of an allergen reduction target. In the allergen reducing method according to the present invention, it is preferable that by spraying the above-mentioned spray agent in the allergen reduction target space, the allergen reducing composition is caused to be present in the allergen reduction target space. In addition, in the allergen reducing method according to the present invention, it is preferable that by causing the above-mentioned surface treatment agent to be present on the surface of the allergen reduction target, the allergen reducing composition is caused to be present on the surface of the allergen reduction target. The allergen reducing methods according to the present invention do not encompass a method of treatment of the human or animal body.

In addition, a textile structure is described herein which is a textile structure being processed by any of the above-mentioned allergen reducing compositions. In addition, an architectural interior material is described herein which is an architectural interior material being processed by any of the above-mentioned allergen reducing compositions.

### ADVANTAGEOUS EFFECTS OF THE INVENTION

Thus, it is made possible to provide an allergen reducing composition which is capable of reducing allergens such as mites and cedar pollen and of imparting a function of reducing allergens to unwoven fabric, textile, or a textile product and which causes less coloring of an allergen reduction target; a spray agent using the allergen reducing composition; a surface treatment agent using the allergen reducing composition; and an allergen reducing method.

### DESCRIPTION OF EMBODIMENT

In the present invention, a zinc salt of gluconic acid, citric acid, lactic acid or malic acid is used. Among these, the gluconic acid, the citric acid, and the lactic acid are preferable and further, the gluconic acid is more preferable. For example, zinc gluconate exhibits extremely high safety with a value of acute oral toxicity in a rat which exceeds 5,000 mg/kg and is used as a breast milk substitutional food and a health functional food aiming at the prevention and treatment of a zinc deficiency.

In the present invention, copper gluconate is used as a supplement and a health functional food of copper.

As a rare earth salt used in the present invention, cited is scandium salt, yttrium salt, lanthanum salt, cerium salt, praseodymium salt, neodymium salt, samarium salt, europium salt, gadolinium salt, terbium salt, dysprosium salt, holmium salt, erbium salt, thulium salt, ytterbium salt, or lutetium salt. In particular, the lanthanum salt, the cerium salt, the erbium salt, and the ytterbium salt are preferable, and the lanthanum salt and the cerium salt are more preferable. These rare earth salts which are commercially available as reagents and industrial raw materials in general can be used.

As a salt, any of salts each having counter ions can be used. A chloride salt, a sulfate salt, a nitrate salt, a bromide salt, an iodide salt, a carbonate salt, a phosphoric salt, and an organic acid salt can be exemplified. As the organic acid salt, a salt of monovalent or divalent carboxylic acid and a salt of hydroxy acid are cited. Specifically, as the hydroxy acid, the lactic acid, the gluconic acid, the tartaric acid, the citric acid, and the malic acid can be exemplified, and as the monovalent or divalent carboxylic acid, acetic acid, propionic acid, malonic acid, succinic acid, glutaric acid, adipic acid, and azelaic acid can be exemplified. Among these, the chloride salt and the salt of hydroxy acid are preferable, and further, the chloride salt, the gluconic acid salt, and the lactic acid salt are more preferable.

The rare earth salt of the hydroxy acid which is commercially available as a reagent and an industrial raw material in general can be used. In addition, the rare earth salt of the hydroxy acid can also be prepared from the hydroxy acid and a general-purpose inorganic rare earth salt, or a rare earth oxide. As the materials used for preparing the rare earth salt of the hydroxy acid, materials which are commercially available as reagents and industrial raw materials in general can be used. For the preparation, a method of directly reacting the rare earth oxide to an aqueous solution of the hydroxy acid is cited. In addition, the rare earth salt of the hydroxy acid can also be prepared by reacting a hydroxide of alkali metal to an aqueous solution of the inorganic rare earth salt such as chloride and thereby previously preparing hydroxide of the rare earth salt and further, by reacting the hydroxy acid to the hydroxide and thereby preparing the rare earth salt of hydroxy acid. As the hydroxide of the alkali metal, for example, sodium hydroxide, potassium hydroxide, and lithium hydroxide is cited, and the sodium hydroxide is preferable.

As the allergens which the allergen reducing composition according to the present invention targets, cited are allergens (Der p1) derived from feces of mites "Dermatophagoides pteronyssinus"; allergens (Der p2) derived from bodies of the mites "Dermatophagoides pteronyssinus"; allergens (Der f1) derived from feces of mites "Dermatophagoides farinae"; and allergens (Der f2) derived from bodies of the mites "Dermatophagoides farinae". In addition, as allergens derived from plants, first cited are allergens (Cry j1) derived from pollen of cedars "Cryptomeria japonica", and further cited are allergens derived from pollen of hinoki cypresses, ragweeds, mugworts, orchard grass, and sweet vernal grass. In addition thereto, cited are allergens (Can f1 and Can f2) derived from hairs and dandruff of dogs; allergens (Fel d1) derived from hairs and dandruff of cats; allergens (Bra g1 and Bra g2) derived from cockroaches; allergens (Alt a1) derived from mold; allergens derived from a natural rubber latex, and like. Among these, great importance is attached to the allergens derived from the mites and the allergens derived from the cedar pollen as the allergens which particularly need to be reduced. By using the composition according to the present invention, and the unwoven fabric, the textile, or the textile product, or the architectural interior material which are processed with the composition according to the present invention, the variety of allergens as cited above can be reduced, and the many kinds of allergens can be substantially eliminated.

By causing the allergen reducing composition according to the present invention to be present on a surface of an allergen reduction target as an environment in which allergens are present, the allergens of the allergen reduction target can be reduced. As the allergen reduction target, for example, there are unwoven fabric, textile, a textile product, and an architectural interior material. It is noted that these are examples of the allergen reduction targets, and the allergen reduction target are not limited thereto. Each of the unwoven fabric, the textile, and the textile product is one example of a textile structure.

In a case where the allergen reduction target is the textile structure such as the unwoven fabric, the textile, or the textile product, the allergen reducing composition is caused to be present on the surface of the allergen reduction target by employing a method of immersing the surface of the allergen reduction target in the allergen reducing composition, applying the allergen reducing composition to the surface of the allergen reduction target, or spraying the allergen reducing composition to the surface of the allergen reduction target, or others. As the textile or the textile product, cited are clothing, interior commodities such as a carpet, a sofa, wall paper, and a curtain, bedding such as Futon ticking, a Futon cover, Futon padding, a sheet, a pillow cover, and a mat, automotive parts such as a car seat and a car mat, and stuffed toys. As the unwoven fabric, cited are a cleaning wet wiper, a mask, a filter material, and a dust bag of an electric vacuum cleaner.

There are a variety of the unwoven fabric and the textile which can be processed by using the allergen reducing composition according to the present invention. For example, nylon, cotton, polyester, and wool, are cited, and composite fiber in which two or more of these kinds of fiber are used can also be processed thereby. In addition, the allergen reducing composition according to the present invention can also be used for unwoven fabric using polyethylene or polypropylene. Although a method of processing or treating of the allergen reducing composition according to the present invention to the unwoven fabric, the textile, or the textile product is not particularly limited, immersion treatment, spray treatment, or uptake processing can be conducted.

In addition, in a case where the allergen reduction target is an architectural interior material, surface treatment for the architectural interior material is conducted by using a coating agent, or paint as one example of a surface treatment agent containing the allergen reducing composition, thereby causing the allergen reducing composition to be present on a surface of the architectural interior material. As the architectural interior material, cited are wall paper using resin such as vinyl chloride, a ceiling material, a wooden material such as plywood, and a floor material.

In a case where the allergen reducing composition according to the present invention is applied to these architectural interior materials, it is preferable to employ a method of the surface treatment. In particular, by using a surface treatment agent obtained by adding the allergen reducing composition according to the present invention to a water-soluble or aqueous surface treatment agent or coating agent, effective surface treatment can be conducted. In addition, as for the paint, it is preferable to add the allergen reducing composition to aqueous paint.

An aqueous surface treatment agent which is commercially available as an industrial raw material in general can be used. This sort of the surface treatment agent is not particularly limited. Starch, a variety of modified starches such as an enzymatically modified starch, and a water-soluble polymer such as polyvinyl alcohol, polyacrylamide, and carboxymethyl cellulose are cited. In addition, cited is an aqueous resin emulsion such as an acrylic resin emulsion, a vinyl acetate resin emulsion, an acrylic-styrene resin emulsion, an acrylic-vinyl acetate resin emulsion, a vinyl chloride resin emulsion, a vinyl chloride-acrylic resin emulsion, and a urethane resin emulsion. As the allergen reducing wall paper according to the present invention, using the aqueous resin emulsion from which water resistance of cured film can be expected is preferable, rather than using the water-soluble polymer. The wording "being aqueous" indicates that a polymer contained in the surface treatment agent is dissolved in water or a water-insoluble polymer is dispersed in water or a polar solvent in the form of the resin emulsion. This sort of the aqueous surface treatment agent can be mixed macroscopically evenly with water at an arbitrary ratio.

For example, as the acrylic resin emulsion, VONCOAT (a registered trademark, manufactured by DIC Corporation) is cited; as the vinyl chloride resin emulsion or a copolymer resin emulsion of the vinyl chloride-acrylic ester or -vinyl acetate, Vinyblan (a registered trademark, manufactured by Nissin Chemical Industry CO., Ltd) is cited; and as the urethane emulsion, SUPERFLEX (a registered trademark, manufactured by Dai-ichi Kogyo Seiyaku), and ADEKA BONTIGHTER HUX (a registered trademark, manufactured by ADEKA CORPORATION) are cited.

In a case where for the above-described applications, processing using the allergen reducing composition according to the present invention is conducted, in order to facilitate the processing, the allergen reducing composition can be made to be a preparation suited for the processing. As such a preparation, cited are a water-soluble agent in which the allergen reducing composition is dissolved in water or a water-soluble solvent; an oil solution or emulsion in which solubility of the allergen reducing composition in a water-insoluble agent is enhanced; and a powder agent or a granular agent in which the allergen reducing composition is held by an appropriate carrier. Among these, the water-soluble agent dissolved in the water or the water-soluble solvent is preferable.

In addition, in a case where the allergen reducing composition according to the present invention is applied as a spray agent, a water-based liquid agent is suited. Such an agent is prepared such that the allergen reducing composition is caused to contain a zinc salt or a copper salt and a rare earth salt in a range of 0.01% to 20% as a total of active ingredients; it is preferable that the allergen reducing composition contains the zinc salt or the copper salt and the rare earth salt in a range of 0.1% to 5.0% as the total of active ingredients; and it is further preferable that the allergen reducing composition contains the zinc salt or the copper salt and the rare earth salt in a range of 0.1% to 2% as the total of active ingredients. In addition, in order to enhance dryability upon spraying, a volatile organic solvent can be added. Although such an organic solvent is not particularly limited, as a solvent having no problem in safety and having high volatility, ethanol is cited. Further, as other components, an aroma chemical, a deodorant component, or an antimicrobial component can be added to the spray agent.

In addition, in a case where the allergen reducing composition according to the present invention is applied as a surface treatment agent, the zinc salt or the copper salt and the rare earth salt in a range of 0.5% to 70% as a total of active ingredients can be contained therein; preferably, the zinc salt or the copper salt and the rare earth salt in a range of 1% to 50% as the total of active ingredients can be contained therein; and further preferably, the zinc salt or the copper salt and the rare earth salt in a range of 2% to 30% as the total of active ingredients can be contained therein. It is preferable that a mixing ratio of the zinc salt or the copper salt and the rare earth salt is 1 : 9 to 9 : 1 in a weight ratio.

The above-mentioned spray agent can be applied to a residential space such as a room inside and a space in a vehicle such as an automobile and a train as an environment where the allergens are present. A variety of allergens such as mites and pollen which are present so as to adhere to the spaces or to be suspended therewithin can be reduced. Each of the residential space such as the room inside and the space in the vehicle is one example of a space of the allergen reduction target.

In conducting the preparation, for the purpose of enhancing stability, a surfactant can be added. The surfactant is not particularly limited, and a non-ionic surfactant, an anionic surfactant, a cationic surfactant, and an amphoteric surfactant can be cited. Although a kind of the non-ionic surfactant is not particularly limited, for example, cited are polyoxyethylene alkyl phenyl ether, polyoxyethylene styrylphenyl ether, polyoxyethylene alkyl ether, polyoxyethylene alkenyl ether, sorbitan fatty acid ester, and polyoxyethylene sorbitan fatty acid ester. As the anionic surfactant, cited are alkylbenzene sulfonate, polyoxyethylene alkyl phenyl ether sulfate, and dialkyl sulfosuccinate. As the cationic surfactant, cited are an alkylamine salt and a quaternary ammonium salt thereof. As the amphoteric surfactant, cited are a betaine type amphoteric surfactant and an aminocarboxylate salt. In addition, each of these non-ionic surfactant, anionic surfactant, cationic surfactant, amphoteric surfactant may be singly used, or two kinds or more of these surfactants may be used in combination.

An allergen reducing component which has been publicly known can be added to the allergen reducing composition according to the present invention in a range in which physical properties thereof are not impaired. As the allergen reducing component, cited are a hydroxybenzoic acid-based compound such as dihydroxybenzoic acid and 2, 4, 6-trihydroxybenzoic acid or a salt thereof, polystyrene-based compound such as para-hydroxy polystyrene and a polystyrene sulfonate salt, a natural extract such as persimmon tannin, and an inorganic salt-based compound such as a calcium salt and a strontium salt.

In a case where for the purpose of removing the allergens which are house dust mites, the allergen reducing composition according to the present invention is used, a miticide is concurrently used in conjunction therewith, thereby allowing an allergen reducing effect thereof to be further sustained. The miticide to be used is not particularly limited, and it is only required for the miticide to have a lethal effect or a repellent effect against the house dust mites. For example, benzyl alcohol, benzyl benzoate, phenyl salicylate, cinnamaldehyde, a hyssop oil, or a carrot seed oil can be used. In addition, a pyrethroid-based compound such as natural pyrethrin, phenothrin, and permethrin, an organic phosphorous compound such as fenitrothion, malathion, fenthion, and diazinon, dicofol, chlorobenzilate, hexythiazox, tebufenpyrad, pyridaben, or amidoflumet, can be used.

There may be a case where in connection with the allergen reducing composition according to the present invention, the growth of mold or bacteria is of concern. Therefore, a fungicidal agent or an antibacterial agent can be concurrently used in conjunction therewith. A kind of the fungicidal agent or the antibacterial agent is not particularly limited, and it is only required for the fungicidal agent or the antibacterial agent to have a fungicidal activity or an antibacterial activity. For example, 5-chloro-N-methylisothiazolone; methylenebisthiocyanate; 2-bromo-2-nitropropane-1, 3-diol; glutaraldehyde; iodo-propynylbutyl carbamate; a zinc salt of pyridinethiol-N-oxide; 1,2-benzoisothiazolone; 1,2-dibromo-2,4-dicyanobutane; chlorhexidine gluconate; 2-isopropyl-5-methylphenol; 3-methyl-4-isopropylphenol; orthophenyl phenol; methyl parahydroxybenzoate; ethyl parahydroxybenzoate; propyl parahydroxybenzoate; butyl parahydroxybenzoate; parachlorometaxylenol; parachlorometacresol; polylysine; benzalkonium chloride; didecyldimethyl ammonium chloride; N-n-butyl-benzoisothiazolone; N-octylisothiazolone; 2-(4-thiazolyl) benzimidazole; methyl 2-benzimidazolyl carbamate; tetrachloroisophthalonitrile; diiodomethyl-para-tolylsulfone; para-chlorophenyl-3-iodopropargylformal; 2,3,5,6-tetrachloro-4-(methyl sulfonyl) pyridine; fatty acid glycerin ester; or hinokitiol can be used.

Upon preparing the allergen reducing composition according to the present invention, in addition to the above-described surfactant, miticide, and antibacterial agent, as needed, a chelate agent, a corrosion inhibitor, an aroma chemical, a scale inhibitor, a defoaming agent, an antistatic agent, a resin binder, a thickening agent, or a softening treatment agent can be added.

As a form in which the allergen reducing composition according to the present invention is used, an application for a spray agent and an application for a processing agent are cited. The allergen reducing composition is added to a composition with which people are likely to come in contact in the environment, for example, a softening agent, a deodorant, a fungicidal agent, a sterilizing agent, a pesticide, paint, or an adhesive, thereby also allowing allergens in the environment to be reduced. A material with which people are likely to come in contact in the environment, for example, an architectural material such as a wooden material, concrete, metal, stone, or glass; or a molded product formed of rubber, paper, resin, or plastic is processed by the allergen reducing composition according to the present invention, thereby also allowing the allergens in the environment to be reduced.

### [Examples]

The present invention will be described in a further detailed manner with reference to Examples and Test Examples. However, the present invention is not limited by these. It is noted that % shown below all indicates a weight percent.

For the preparation of Examples and Comparative Examples, the following reagents were used as raw materials.

Lanthanum chloride heptahydrate (manufactured by Wako Pure Chemical Industries, Ltd.)

Cerium chloride heptahydrate (manufactured by Wako Pure Chemical Industries, Ltd.)

Samarium chloride hexahydrate (manufactured by Wako Pure Chemical Industries, Ltd.)

Gadolinium chloride hexahydrate (manufactured by Wako Pure Chemical Industries, Ltd.)

Dysprosium chloride hexahydrate (manufactured by Wako Pure Chemical Industries, Ltd.)

Holmium chloride hexahydrate (manufactured by Wako Pure Chemical Industries, Ltd.)

Erbium chloride hexahydrate (manufactured by Wako Pure Chemical Industries, Ltd.)

Ytterbium chloride hexahydrate (manufactured by Wako Pure Chemical Industries, Ltd.)

Ytterbium nitrate tetrahydrate (manufactured by Wako Pure Chemical Industries, Ltd.)

Lanthanum oxide (manufactured by Wako Pure Chemical Industries, Ltd.)

Neodymium oxide (manufactured by Wako Pure Chemical Industries, Ltd.)

Zinc gluconate (Helshas-Zn (a registered trademark), manufactured by FUSO CHEMICAL CO., LTD.)

Copper gluconate (manufactured by Wako Pure Chemical Industries, Ltd.)

Zinc chloride (manufactured by Wako Pure Chemical Industries, Ltd.)

Copper sulfate pentahydrate (manufactured by Wako Pure Chemical Industries, Ltd.)

Calcium chloride dihydrate (manufactured by Wako Pure Chemical Industries, Ltd.)

Strontium chloride hexahydrate (manufactured by Wako Pure Chemical Industries, Ltd.)

Aluminum chloride hexahydrate (manufactured by Wako Pure Chemical Industries, Ltd.)

Gallium nitrate n-hydrate (n = 7 to 9)(manufactured by Wako Pure Chemical Industries, Ltd.)

Indium chloride tetrahydrate (manufactured by Kisan Kinzoku Chemicals Co., Ltd.)

Cobalt chloride hexahydrate (manufactured by OUTKUMPU)

Nickel chloride hexahydrate (manufactured by Wako Pure Chemical Industries, Ltd.)

Basic zirconium chloride (approximately 45%)(Zircosol ZC-2 (a registered trademark), manufactured by DAIICHI KIGENSO KAGAKU KOGYO Co., LTD.)

90% lactic acid (manufactured by Musashino Chemical Laboratory, Ltd.)

Malic acid (DL-malic acid, manufactured by Wako Pure Chemical Industries, Ltd.)

50% gluconic acid aqueous solution (manufactured by Wako Pure Chemical Industries, Ltd.)

Tannic acid (manufactured by Fuji Chemical Industries Co., Ltd.)

### Preparation of lanthanum lactate aqueous solution

In a conical flask having a capacity of 100 mL to which an Allihn condenser tube was attached, 4.5 g of a 90% lactic acid solution and 69.0 g of ion exchanged water were mixed and further, 1.2g of lanthanum oxide was added thereto. The resultant was warmed in a hot water bath at up to 75°C and agitation was continued for three hours, thereby dissolving the lanthanum oxide. The resultant was cooled, thereby obtaining an aqueous solution whose content of lanthanum lactate was approximately 4%.

### Preparation of neodymium lactate aqueous solution

In a conical flask having a capacity of 100 mL to which an Allihn condenser tube was attached, 10.4 g of a 90% lactic acid solution and 29.4 g of ion exchanged water were mixed and further, 4.2g of neodymium oxide was added thereto. The resultant was warmed in a hot water bath at up to 75°C and agitation was continued for one hour and thereafter, one drop of sulfuric acid was added thereto. Further, the agitation was continued for one hour, thereby dissolving the neodymium oxide. After cooling, the resultant was taken out and was diluted by using ion exchanged water so as to allow a total amount to be 120 g, thereby obtaining a purple aqueous solution whose content of neodymium lactate was approximately 4%.

### Preparation of neodymium gluconate aqueous solution

In a conical flask having a capacity of 500 mL to which an Allihn condenser tube was attached, 20.5 g of a 50% gluconic acid aqueous solution and 300 g of ion exchanged water were mixed and further, 2.2 g of neodymium oxide was added thereto. The resultant was warmed in a hot water bath at up to 75°C and agitation was continued for one hour and thereafter, one drop of sulfuric acid was added thereto. Further, the agitation was continued for three hours, and the resultant was diluted by using ion exchanged water so as to allow a total amount to be 480 g, thereby dissolving the neodymium oxide. The resultant was cooled, thereby obtaining a purple aqueous solution whose content of neodymium gluconate was approximately 2% (Comparative Example 24).

### Preparation of lanthanum malate aqueous solution

In a conical flask having a capacity of 300 mL to which an Allihn condenser tube was attached, 3.1 g of malic acid and 200 g of ion exchanged water were mixed and further, 1.2 g of lanthanum oxide was added thereto. The resultant was warmed in a hot water bath at up to 75°C and agitation was continued for two hours and thereafter, one drop of sulfuric acid was added thereto. Further, the agitation was continued for three hours, thereby dissolving the lanthanum oxide. The resultant was cooled, thereby obtaining an aqueous solution whose content of lanthanum malate was approximately 2% (Comparative Example 39).

### Preparation of neodymium malate aqueous solution

In a conical flask having a capacity of 300 mL to which an Allihn condenser tube was attached, 3.1 g of malic acid and 200 g of ion exchanged water were mixed and further, 1.2 g of neodymium oxide was added thereto. The resultant was warmed in a hot water bath at up to 75°C and agitation was continued for two hours and thereafter, one drop of sulfuric acid was added thereto. Further, the agitation was continued for three hours, thereby dissolving the neodymium oxide. The resultant was cooled, thereby obtaining a purple aqueous solution whose content of neodymium malate was approximately 2% (Comparative Example 40).

Examples 1 to 24 were prepared with blending ratios shown in Tables 1 to 3, and Comparative Examples 1 to 46 were prepared with blending ratios shown in Tables 4 to 9.

**[Table 1]**

| | Example | | | | | | | |
|---|---|---|---|---|---|---|---|---|
| | 1 | 2 | 3 | 4 | 5 | 6 | 7 | 8 |
| Zinc gluconate | 1.0 | 1.0 | 1.0 | 1.0 | 1.0 | 1.0 | 1.0 | 1.0 |
| Lanthanum chloride heptahydrate | 1.0 | - | - | - | - | - | - | - |
| Cerium chloride heptahydrate | - | 1.0 | - | - | - | - | - | - |
| Samarium chloride hexahydrate | - | - | 1.0 | - | - | - | - | - |
| Gadolinium chloride hexahydrate | - | - | - | 1.0 | - | - | - | - |
| Dysprosium chloride hexahydrate | - | - | - | - | 1.0 | - | - | - |
| Holmium chloride hexahydrate | - | - | - | - | - | 1.0 | - | - |
| Erbium chloride hexahydrate | - | - | - | - | - | - | 1.0 | - |
| Ytterbium chloride hexahydrate | - | - | - | - | - | - | - | 1.0 |
| Ion exchanged water | 98.0 | 98.0 | 98.0 | 98.0 | 98.0 | 98.0 | 98.0 | 98.0 |

**[Table 2]**

| | Example | | | | | | | |
|---|---|---|---|---|---|---|---|---|
| | 9 | 10 | 11 | 12 | 13 | 14 | 15 | 16 |
| Zinc gluconate | 1.0 | - | - | - | - | - | - | - |
| Zinc chloride | - | 0.25 | 0.25 | 0.25 | 0.25 | - | - | - |
| Copper gluconate | - | - | - | - | - | 0.5 | 0.5 | 0.5 |
| Ytterbium nitrate tetrahydrate | 1.0 | - | - | - | - | - | - | - |
| Lanthanum chloride heptahydrate | - | 1.0 | - | - | - | 1.0 | - | - |
| Erbium chloride hexahydrate | - | - | 1.0 | - | - | - | 1.0 | - |
| Ytterbium chloride hexahydrate | - | - | - | 1.0 | - | - | - | 1.0 |
| Gadolinium chloride hexahydrate | - | - | - | - | 1.0 | - | - | - |
| Ion exchanged water | 98.0 | 98.75 | 98.75 | 98.75 | 98.75 | 98.5 | 98.5 | 98.5 |

**[Table 3]**

| | Example | | | | | | | |
|---|---|---|---|---|---|---|---|---|
| | 17 | 18 | 19 | 20 | 21 | 22 | 23 | 24 |
| Zinc gluconate | - | 1.0 | 1.0 | 1.0 | 1.0 | 1.0 | 1.0 | - |
| Zinc chloride | - | - | - | - | - | - | - | 0.25 |
| Copper sulfate pentahydrate | 0.5 | - | - | - | - | - | - | - |
| Copper gluconate | - | - | - | - | - | - | 0.25 | - |
| Lanthanum chloride heptahydrate | 1.0 | - | - | - | - | - | 0.5 | - |
| Lanthanum lactate aqueous solution | - | 25.0 | - | - | - | - | - | - |
| Neodymium lactate aqueous solution | - | - | 25.0 | - | - | - | - | 25.0 |
| Neodymium gluconate aqueous solution | - | - | - | 50.0 | - | - | - | - |
| Lanthanum malate aqueous solution | - | - | - | - | 50.0 | - | - | - |
| Neodymium malate aqueous solution | - | - | - | - | - | 50.0 | - | - |
| Ion exchanged water | 98.5 | 74.0 | 74.0 | 49.0 | 49.0 | 49.0 | 98.25 | 74.75 |

**[Table 4]**

| | Comparative Example | | | | | | | |
|---|---|---|---|---|---|---|---|---|
| | 1 | 2 | 3 | 4 | 5 | 6 | 7 | 8 |
| Lanthanum chloride heptahydrate | 2.0 | - | - | - | - | - | - | - |
| Cerium chloride heptahydrate | - | 2.0 | - | - | - | - | - | - |
| Samarium chloride hexahydrate | - | - | 2.0 | - | - | - | - | - |
| Gadolinium chloride hexahydrate | - | - | - | 2.0 | - | - | - | - |
| Dysprosium chloride hexahydrate | - | - | - | - | 2.0 | - | - | - |
| Holmium chloride hexahydrate | - | - | - | - | - | 2.0 | - | - |
| Erbium chloride hexahydrate | - | - | - | - | - | - | 2.0 | - |
| Ytterbium chloride hexahydrate | - | - | - | - | - | - | - | 2.0 |
| Ion exchanged water | 98.0 | 98.0 | 98.0 | 98.0 | 98.0 | 98.0 | 98.0 | 98.0 |

**[Table 5]**

| | Comparative Example | | | | | | | |
|---|---|---|---|---|---|---|---|---|
| | 9 | 10 | 11 | 12 | 13 | 14 | 15 | 16 |
| Ytterbium nitrate tetrahydrate | 2.0 | - | - | - | - | - | - | - |
| Zinc gluconate | - | 2.0 | - | - | - | - | - | - |
| Zinc chloride | - | - | 0.5 | - | - | - | - | - |
| Copper gluconate | - | - | - | 1.0 | - | - | - | - |
| Copper sulfate pentahydrate | - | - | - | - | 1.0 | - | - | - |
| Calcium chloride dihydrate | - | - | - | - | - | 2.0 | - | - |
| Strontium chloride hexahydrate | - | - | - | - | - | - | 2.0 | - |
| Aluminum chloride hexahydrate | - | - | - | - | - | - | - | 2.0 |
| Ion exchanged water | 98.0 | 98.0 | 99.5 | 99.0 | 99.0 | 98.0 | 98.0 | 98.0 |

**[Table 6]**

| | Comparative Example | | | | | | | |
|---|---|---|---|---|---|---|---|---|
| | 17 | 18 | 19 | 20 | 21 | 22 | 23 | 24 |
| Gallium nitrate n-hydrate (n = 7 to 9) | 2.0 | - | - | - | - | - | - | - |
| Indium chloride tetrahydrate | - | 2.0 | - | - | - | - | - | - |
| Cobalt chloride hexahydrate | - | - | 1.0 | - | - | - | - | - |
| Nickel chloride hexahydrate | - | - | - | 1.0 | - | - | - | - |
| Basic zirconium chloride (approx. 45%) | - | - | - | - | 4.0 | - | - | - |
| Lanthanum lactate aqueous solution | - | - | - | - | - | 50.0 | - | - |
| Neodymium lactate aqueous solution | - | - | - | - | - | - | 50.0 | - |
| Neodymium gluconate aqueous solution | - | - | - | - | - | - | - | 100 |
| Ion Exchanged water | 98.0 | 98.0 | 99.0 | 99.0 | 96.0 | 50.0 | 50.0 | - |

**[Table 7]**

| | Comparative Example | | | | | | | |
|---|---|---|---|---|---|---|---|---|
| | 25 | 26 | 27 | 28 | 29 | 30 | 31 | 32 |
| Lanthanum chloride heptahydrate | 1.0 | 1.0 | 1.0 | 1.0 | 1.0 | 1.0 | 1.0 | 1.0 |
| Calcium chloride dihydrate | 1.0 | - | - | - | - | - | - | - |
| Strontium chloride hexahydrate | - | 1.0 | - | - | - | - | - | - |
| Aluminum chloride hexahydrate | - | - | 1.0 | - | - | - | - | - |
| Gallium nitrate n-hydrate (n = 7 to 9) | - | - | - | 1.0 | - | - | - | - |
| Indium chloride tetrahydrate | - | - | - | - | 1.0 | - | - | - |
| Cobalt chloride hexahydrate | - | - | - | - | - | 0.5 | - | - |
| Nickel chloride hexahydrate | - | - | - | - | - | - | 0.5 | - |
| Basic zirconium chloride (approx. 45%) | - | - | - | - | - | - | - | 2.0 |
| Ion exchanged water | 98.0 | 98.0 | 98.0 | 98.0 | 98.0 | 98.5 | 98.5 | 97.0 |

**[Table 8]**

| | Comparative Example | | | | | | | |
|---|---|---|---|---|---|---|---|---|
| | 33 | 34 | 35 | 36 | 37 | 38 | 39 | 40 |
| Zinc gluconate | 1.0 | 1.0 | 1.0 | 1.0 | 1.0 | 1.0 | - | - |
| Calcium chloride dihydrate | 1.0 | - | - | - | - | - | - | - |
| Strontium chloride hexahydrate | - | 1.0 | - | - | - | - | - | - |
| Aluminum chloride hexahydrate | - | - | 1.0 | - | - | - | - | - |
| Cobalt chloride hexahydrate | - | - | - | 0.5 | - | - | - | - |
| Nickel chloride hexahydrate | - | - | - | - | 0.5 | - | - | - |
| Basic zirconium chloride (approx. 45%) | - | - | - | - | - | 2.0 | - | - |
| Lanthanum malate aqueous solution | - | - | - | - | - | - | 100 | - |
| Neodymium malate aqueous solution | - | - | - | - | - | - | - | 100 |
| Ion exchanged water | 98.0 | 98.0 | 98.0 | 98.5 | 98.5 | 97.0 | - | - |

**[Table 9]**

| | Comparative Example | | | | | |
|---|---|---|---|---|---|---|
| | 41 | 42 | 43 | 44 | 45 | 46 |
| Copper gluconate | 0.5 | 0.5 | - | - | - | - |
| Calcium chloride dihydrate | 1.0 | - | - | - | - | - |
| Strontium chloride hexahydrate | - | 1.0 | - | - | - | - |
| Lanthanum chloride heptahydrate | - | - | 1.0 | 1.0 | - | - |
| Erbium chloride hexahydrate | - | - | 1.0 | - | - | - |
| Ytterbium chloride hexahydrate | - | - | - | 1.0 | - | - |
| Zinc gluconate | - | - | - | - | - | 1.0 |
| Tannic acid | - | - | - | - | 2.0 | 1.0 |
| Ion exchanged water | 98.5 | 98.5 | 98.0 | 98.0 | 98.0 | 98.0 |

### [Test Example 1]

### Measurement of effect of reducing mite allergen by the allergen reducing composition

Against an allergen liquid containing mite allergen Der f2, whose quantity of protein was approximately 900 ng/1mL {a phosphate buffer solution (pH7.2)}, 25 µL of each of Examples 1 to 24 and each of Comparative Examples 1 to 46 was reacted. With respect to these samples, effect of reducing mite allergens was measured by employing a sandwich method of a Der f2 enzyme-linked immunosorbent assay (ELISA). First, anti-Der f2 monoclonal antibody 15E11, which was diluted with a phosphate buffer solution (pH7.4) to be at 2 µg/mL, were added thereto, with 100 µL thereof added into each well of a F16 MAXISORP NUNC-IMMUNO MODULE plate (manufactured by Nalge Nunc International). Coating of antibody was conducted at 4°C for three days or more. After the coating, the liquid was removed, and a blocking reagent {1 wt.% bovine serum albumin + phosphate buffer solution (pH7.2)} was added, with 200 µL thereof into each well, and reacted at 37°C for 60 minutes. After the reaction, the plate was rinsed with a phosphate buffer solution (pH7.2).

Then, the extraction liquid obtained by reacting the mite allergen and the above-mentioned composition was dropped, with 100 µL being dropped into each well, and reacted at 37°C for 60 minutes. After the reaction, the plate was rinsed with the phosphate buffer solution (pH7.2). Anti-Der f2 monoclonal antibody labeled by peroxidase was dissolved in a phosphate buffer solution {pH7.2, containing 1 wt.% bovine serum albumin and 0.05 wt.% polyoxyethylene (20) sorbitan monolaurate} to be at 200 µg/mL, and the resultant was diluted by 1,200 times with the phosphate buffer solution (pH7.2, containing 1 wt.% bovine serum albumin and 0.05 wt.% polyoxyethylene (20) sorbitan monolaurate). The resultant liquid was added, with 100 µL thereof into each well, and reacted at 37°C for 60 minutes. After the reaction, the plate was rinsed with the phosphate buffer solution (pH7.2). One tablet of ortho-phenylenediamine dihydrochloride (13 mg Tablet, manufactured by Wako Pure Chemical Industries, Ltd.) and 6.5 µL of a 30% hydrogen peroxide solution were added to 6.5 mL of a 0.1 mol/L phosphate buffer solution (pH6.2). The resultant was added, with 100 µL thereof to each well. The reaction was caused at 37°C for three minutes. Immediately after the reaction, 50 µL of 1 mol/L H₂SO₄ was added into each well, thereby stopping the reaction. By using a microplate spectrophotometer (manufactured by Tecan Japan Co., Ltd.), an absorbance (OD at 490 nm) was measured. Results are shown in Tables 10 to 12.

**[Table 10]**

| Sample | Der f2 concentration (ng/mL) | Reduction ratio (%) | Sample | Der f2 concentration (ng/mL) | Reduction ratio (%) |
|---|---|---|---|---|---|
| Example 1 | 52 | 94 | Example 13 | 200 | 78 |
| Example 2 | 66 | 93 | Example 14 | 170 | 81 |
| Example 3 | 100 | 89 | Example 15 | 180 | 80 |
| Example 4 | 95 | 89 | Example 16 | 180 | 80 |
| Example 5 | 110 | 88 | Example 17 | 160 | 82 |
| Example 6 | 88 | 90 | Example 18 | 66 | 93 |
| Example 7 | 57 | 94 | Example 19 | 210 | 77 |
| Example 8 | 78 | 91 | Example 20 | 82 | 91 |
| Example 9 | 79 | 91 | Example 21 | 190 | 79 |
| Example 10 | 200 | 78 | Example 22 | 170 | 81 |
| Example 11 | 100 | 89 | Example 23 | 200 | 78 |
| Example 12 | 73 | 92 | Example 24 | 130 | 86 |

**[Table 11]**

| Sample | Der f2 concentration (ng/mL) | Reduction ratio (%) | Sample | Der f2 concentrat ion (ng/mL) | Reduction ratio (%) |
|---|---|---|---|---|---|
| Comparative Example 1 | 680 | 24 | Comparative Example 13 | 300 | 67 |
| Comparative Example 2 | 710 | 21 | Comparative Example 14 | 740 | 18 |
| Comparative Example 3 | 510 | 43 | Comparative Example 15 | 760 | 16 |
| Comparative Example 4 | 350 | 61 | Comparative Example 16 | 850 | 6 |
| Comparative Example 5 | 210 | 77 | Comparative Example 17 | 840 | 7 |
| Comparative Example 6 | 200 | 78 | Comparative Example 18 | 490 | 46 |
| Comparative Example 7 | 200 | 78 | Comparative Example 19 | 870 | 3 |
| Comparative Example 8 | 240 | 73 | Comparative Example 20 | 820 | 9 |
| Comparative Example 9 | 270 | 70 | Comparative Example 21 | 750 | 17 |
| Comparative Example 10 | 280 | 69 | Comparative Example 22 | 640 | 29 |
| Comparative Example 11 | 310 | 66 | Comparative Example 23 | 500 | 44 |
| Comparative Example 12 | 280 | 69 | Comparative Example 24 | 560 | 38 |

**[Table 12]**

| Sample | Der f2 concentration (ng/mL) | Reduction ratio (%) | Sample | Der f2 concentration (ng/mL) | Reduction ratio (%) |
|---|---|---|---|---|---|
| Comparative Example 25 | 860 | 4 | Comparative Example 36 | 530 | 41 |
| Comparative Example 26 | 740 | 18 | Comparative Example 37 | 400 | 56 |
| Comparative Example 27 | 740 | 18 | Comparative Example 38 | 770 | 14 |
| Comparative Example 28 | 870 | 3 | Comparative Example 39 | 590 | 34 |
| Comparative Example 29 | 540 | 40 | Comparative Example 40 | 690 | 23 |
| Comparative Example 30 | 670 | 26 | Comparative Example 41 | 400 | 56 |
| Comparative Example 31 | 740 | 18 | Comparative Example 42 | 360 | 60 |
| Comparative Example 32 | 690 | 23 | Comparative Example 43 | 480 | 47 |
| Comparative Example 33 | 670 | 26 | Comparative Example 44 | 380 | 58 |
| Comparative Example 34 | 670 | 26 | Comparative Example 45 | 180 | 80 |
| Comparative Example 35 | 620 | 31 | Comparative Example 46 | 740 | 18 |

### [Test Example 2]

### Measurement of effect of reducing cedar pollen allergens by the allergen reducing composition

Against an allergen liquid of an approximately 12.5 ng/1mL {phosphate buffer solution (pH7.2)} as cedar pollen allergens Cry j1, 25 µL of each of Examples 1, 7, 8, 10, 11, 12, 14, 15, 16, 18, 19, and 24 and each of Comparative Example 1, 7, 8, 10, 11, 12, 22, 23, 43, 44, 45, and 46 were reacted. With respect to these samples, effect of reducing cedar pollen was measured by employing a sandwich method of a Cry j 1 enzyme-linked immunosorbent assay (ELISA). First, Cry j1 monoclonal antibody 013, which was diluted with a phosphate buffer solution (pH7.4, containing 0.1 wt.% NaN₃) to be at 2 µg/mL, were added, with 100 µL thereof into each well of a F16 MAXISORP NUNC-IMMUNO MODULE plate (manufactured by Nalge Nunc International). Coating of antibody was conducted at 4°C for one day or more. After the coating, the liquid was rinsed, and a blocking reagent {1 wt.% bovine serum albumin + phosphate buffer solution (pH7.2, containing 0.1 wt.% NaN₃)} was added, with 200 µL thereof into each well, and reacted at 37°C for 60 minutes. After the reaction, the plate was rinsed with a phosphate buffer solution {pH7.2, containing 0.1 wt.% polyoxyethylene (20) sorbitan monolaurate } .

Then, the liquid obtained by reacting the cedar pollen allergen and the above-mentioned composition was dropped, with 100 µL being dropped into each well, and reacted at 37°C for 60 minutes. After the reaction, the plate was rinsed with a phosphate buffer solution {pH7.2, containing 0.1 wt.% polyoxyethylene (20) sorbitan monolaurate}. Cry j1 monoclonal antibody 053 labeled by peroxidase was dissolved in distilled water to be at 200 µg/mL, and the resultant was diluted by 1200 times with the phosphate buffer solution {pH7.2, containing 1 wt.% bovine serum albumin and 0.1 wt.% polyoxyethylene (20) sorbitan monolaurate}. The resultant liquid was added, with 100 µL thereof into each well. After the reaction at 37°C for 60 minutes, the plate was rinsed with the phosphate buffer solution {pH7.2, containing 0.1 wt.% polyoxyethylene (20) sorbitan monolaurate}. Ortho-phenylenediamine dihydrochloride (manufactured by SIGMA CHEMICAL CO.: 26 mg Tablet) and 13 µL of a 30% hydrogen peroxide solution were added to 13 mL of a 0.1 mol/L phosphate buffer solution (pH6.2), and the resultant was added, with 100 µL thereof into each well, and reacted at 37°C for five minutes. Immediately after the reaction, 50 µL of 2 mol/L H₂SO₄ was added into each well, thereby stopping the reaction. By using a microplate spectrophotometer (manufactured by Tecan Japan Co., Ltd.), an absorbance (OD at 490 nm) was measured. Results are shown in Table 13.

**[Table 13]**

| Sample | Cry j1 concentration (ng/mL) | Reduction ratio (%) | Sample | Cry j1 concentration (ng/mL) | Reduction ratio (%) |
|---|---|---|---|---|---|
| Example 1 | 0.28 | 98 | Comparative Example 1 | 10.9 | 13 |
| Example 7 | 0.84 | 93 | Comparative Example 7 | 4.6 | 63 |
| Example 8 | 0.17 | 99 | Comparative Example 8 | 4.2 | 66 |
| Example 10 | 0.39 | 97 | Comparative Example 10 | 7.5 | 40 |
| Example 11 | 0.53 | 96 | Comparative Example 11 | 7.4 | 41 |
| Example 12 | 0.43 | 97 | Comparative Example 12 | 1.0 | 92 |
| Example 14 | 0.58 | 95 | Comparative Example 22 | 9.1 | 27 |
| Example 15 | 0.28 | 98 | Comparative Example 23 | 8.8 | 30 |
| Example 16 | 0.37 | 97 | Comparative Example 43 | 7.8 | 39 |
| Example 18 | 0.38 | 97 | Comparative Example 44 | 6.8 | 46 |
| Example 19 | 0.25 | 98 | Comparative Example 45 | 0.76 | 94 |
| Example 24 | 0.92 | 93 | Comparative Example 46 | 10.7 | 14 |

### [Test Example 3]

Examples 25 to 28 were prepared by mixing a 2% zinc gluconate aqueous solution (Comparative Example 10) and a 2% lanthanum chloride heptahydrate aqueous solution (Comparative Example 1) at ratios of 1 : 9, 3 : 7, 7 : 3, and 9 : 1 as shown in Table 14, respectively. It is noted that Example 1 obtained by mixing those at a ratio of 5 : 5 is also shown in Table 14. With respect to these Examples, by employing the same method as in Test Example 1, effect of reducing the mite allergen Der f2 was measured. Results are shown in Table 14.

**[Table 14]**

| | Comparative Example 1 | Example 25 | Example 26 | Example 1 | Example 27 | Example 28 | Comparative Example 10 |
|---|---|---|---|---|---|---|---|
| 2% zinc gluconate | 0 | 1 | 3 | 5 | 7 | 9 | 10 |
| 2% lanthanum chloride heptahydrate | 10 | 9 | 7 | 5 | 3 | 1 | 0 |
| Der f2 concentration (ng/mL) | 680 | 600 | 190 | 52 | 41 | 100 | 280 |
| Reduction ratio (%) | 24 | 33 | 79 | 94 | 95 | 89 | 69 |

### [Test Example 4]

Examples 29 to 32 were prepared by mixing a 2% zinc gluconate aqueous solution (Comparative Example 10) and a 2% ytterbium chloride hexahydrate aqueous solution (Comparative Example 8) at ratios of 1 : 9, 3 : 7, 7 : 3, and 9 : 1 as shown in Table 15, respectively. It is noted that Example 8 obtained by mixing those at a ratio of 5 : 5 is also shown in Table 15. With respect to these Examples, by employing the same method as in Test Example 1, effect of reducing the mite allergen Der f2 was measured. Results are shown in Table 15.

**[Table 15]**

| | Comparative Example 8 | Example 29 | Example 30 | Example 8 | Example 31 | Example 32 | Comparative Example 10 |
|---|---|---|---|---|---|---|---|
| 2% zinc gluconate | 0 | 1 | 3 | 5 | 7 | 9 | 10 |
| 2% ytterbium chloride hexahydrate | 10 | 9 | 3 | 5 | 3 | 1 | 0 |
| Der f2 concentration (ng/mL) | 240 | 180 | 140 | 78 | 54 | 110 | 280 |
| Reduction ratio (%) | 73 | 80 | 84 | 91 | 94 | 88 | 69 |

### Formulation of a surface treatment agent for vinyl chloride wall paper

### [Example 33]

In ion exchanged water, 4 g of zinc gluconate, 4 g of lanthanum chloride heptahydrate, and 3.5 g of propylene glycol were dissolved so as to allow a total amount to be 100 g.

### [Comparative Example 47]

In ion exchanged water, 8 g of zinc gluconate and 3.5 g of propylene glycol were dissolved so as to allow a total amount to be 100 g.

### [Comparative Example 48]

In ion exchanged water, 8 g of lanthanum chloride heptahydrate and 3.5 g of propylene glycol were dissolved so as to allow a total amount to be 100 g.

### Preparation of a vinyl chloride sheet

For preparation of a vinyl chloride wall paper surface layer, 100 g of a vinyl chloride powder (PQB83, manufactured by Shin Dai-ichi Vinyl Corporation) having an average degree of polymerization of 700; 50 g of diisononyl phthalate (DINP, manufactured by Taoka Chemical Co., Ltd.) as a plasticizing agent; 3 g of ADK STAB (a registered trademark) FL100 (manufactured by ADEKA CORPORATION) as a stabilizing agent; and 7 g of isoparaffin (IP Solvent 2028, manufactured by Idemitsu Kosan Co., Ltd.) were mixed by using an agitator. The prepared surface layer liquid was applied to a A4-size PPC sheet as a base material sheet so as to have approximately 200 g/m² and the sheet was heated at 150°C for one minute, thereby obtaining vinyl chloride wall paper.

### [Test Example 5]

Each of Example 33, Comparative Example 47, and Comparative Example 48 was added to a urethane resin emulsion (SUPERFLEX 500M (a registered trademark), manufactured by Dai-ichi Kogyo Seiyaku) as a surface treatment agent as to have the concentration of 12%, respectively, and the resultant was thoroughly mixed. This surface treatment agent was applied to the above-mentioned vinyl chloride sheet so as to have 10 g/m², and the sheet was dried at room temperature, thereby obtaining allergen reducing wall paper.

A circular sheet having a diameter of 8 cm was cut out from each of these sheets of the allergen reducing wall paper, and the brim with approximately 5 mm of each of the wall paper samples was folded such that the treated face to curve concavely. One mL of a mite allergen aqueous solution (with Der f2 concentration of 900 ng/mL) was spread on the sheet and the same sheet whose brim with approximately 5 mm was folded such that the treated face to curve convexly was covered so as to combine the treated face with the allergen reducing composition each other. In order to prevent volatilization of the allergen aqueous solution, the sample was put into a plastic bag with a zipper and was stored at room temperature for one hour, and 100 µL of the allergen aqueous solution was sucked up by using PIPETMAN. By employing the same method as in Test Example 1, amounts of mite allergens were measured. Results are shown in Table 16.

**[Table 16]**

| Sample | Der f2 concentration (ng/mL) | Reduction (%) |
|---|---|---|
| Example 33 Processed wall paper | 77 | 91 |
| Comparative Example 47 Processed wall paper | 340 | 62 |
| Comparative Example 48 Processed wall paper | 240 | 73 |
| Initial amount | 900 | - |

### [Example 34]

In ion exchanged water, 0.8 g of zinc gluconate, 0.4 g of lanthanum chloride heptahydrate, and 0.8 g of polyethylene glycol (with a molecular weight of 20,000) were dissolved so as to allow a total amount to be 100 g, thereby obtaining a spray agent.

### [Comparative Example 49]

In ion exchanged water, 1.6 g of zinc gluconate and 0.8 g of polyethylene glycol (with a molecular weight of 20,000) were dissolved so as to allow a total amount to be 100 g, thereby obtaining a spray agent.

### [Comparative Example 50]

In ion exchanged water, 0.8 g of lanthanum chloride heptahydrate and 0.8 g of polyethylene glycol (with a molecular weight of 20,000) were dissolved so as to allow a total amount to be 100 g, thereby obtaining a spray agent.

### [Test Example 6]

### Measurement of effect of reducing allergens by spray agents

Each of Example 34, Comparative Example 49, and Comparative Example 50 was put in a trigger type atomizing container, and approximately 20 g thereof was sprayed onto 0.5 m² of a floor carpet in the company office. After leaving four hours, a fine dust collecting device "dust collecting bag" (manufactured by SC Environmental Science Co., Ltd.) was attached to a suction hose of an electric vacuum cleaner (PV-H23 with suction power 240W, manufactured by Hitachi, Ltd.). And dust was sucked and collected for one minute from areas each with 0.5 m² which were an area to which each of Example 34, Comparative Example 49, or Comparative Example 50 was sprayed and an area to which any of examples was not sprayed (Control). The dust collecting bag into which the dust was collected was put in a plastic bag with a zipper, and mite allergen was extracted by using 5 mL of a phosphate buffer solution. From the extraction liquids as samples for measuring mite allergen amounts, the mite allergen amounts were measured by employing the sandwich method of Der f2 enzyme-linked immunosorbent assay (ELISA) as the same method in Test Example 1. Results are shown in Table 17.

**[Table 17]**

| Sample | Collected dust amount (g) | Der f2 amount (µg) | Reduction ratio (%) |
|---|---|---|---|
| Example 34 | 0.12 | 0.5 | 84 |
| Comparative Example 49 | 0.16 | 2.8 | 13 |
| Comparative Example 50 | 0.12 | 1.3 | 59 |
| Control | 0.17 | 3.2 | - |

### [Test Example 7]

### Measurement of effect of reducing allergens with respect to processed unwoven fabric

In ion exchanged water, each of Example 27, Comparative Example 1, and Comparative Example 10 was diluted so as to have a predetermined concentration, and in 50 g of the resultant liquid, 80 g/m² of unwoven fabric (20 cm × 15 cm) made of polypropylene was immersed for one minute, was processed with a pick up ratio of approximately 200%, and thereafter, was dried at 100°C for 15 minutes. The dilution ratio was adjusted so as to be approximately 0.15 g/m² as an active ingredient amount. An area with 5 cm × 5 cm was cut out from each of allergen reducing unwoven fabric and put into a plastic bag with a zipper, and one mL of a mite allergen aqueous solution (containing Der f2, with 900 ng/mL of a protein concentration) or a cedar pollen allergen aqueous solution (Cry j1, with 12.5 ng/mL of a concentration) was added thereto. The solution was stored at room temperature for one hour, and 100 µL of the allergen aqueous solution was collected by using PIPETMAN. By employing the same method as in Test Example 1 or Test Example 2, the mite allergen amounts or the cedar pollen allergen amounts were measured. Results are shown in Table 18 and Table 19.

**[Table 18]**

| Sample | Active ingredient processing amount (g/m²) | Der f2 concentration (ng/mL) | Reduction ratio (%) |
|---|---|---|---|
| Example 27 Processed unwoven fabric | 0.13 | 180 | 80 |
| Comparative Example 1 Processed unwoven fabric | 0.15 | 800 | 11 |
| Comparative Example 10 Processed unwoven fabric | 0.14 | 730 | 19 |
| Initial amount | - | 900 | - |

**[Table 19]**

| Sample | Active ingredient processing amount (g/m²) | Cry j 1 concentration (ng/mL) | Reduction ratio (%) |
|---|---|---|---|
| Example 27 Processed unwoven fabric | 0.13 | 1.5 | 88 |
| Comparative Example 1 Processed unwoven fabric | 0.15 | 10.5 | 16 |
| Comparative Example 10 Processed unwoven fabric | 0.14 | 11.4 | 9 |
| Initial amount | - | 12.5 | - |

### [Test Example 8]

### Coloring test by iron

In a glass bottle having a capacity of 10 mL, 2 mL of each of Examples 1, 14, and 18 and Comparative Example 45 was collected, and further, one iron nail (having a length of 38 mm) was put thereinto. External appearance was observed over time. Results are shown in Table 20. With respect to Comparative Example 45 containing tannic acid, since immediately after the contact with the iron nail, coloring purple was observed. However, with respect to Examples 1, 14, and 18, no coloring on their external appearance was observed.

**[Table 20]**

| Sample | Initial appearance | One hour later | One day later | Three days later |
|---|---|---|---|---|
| Example 1 | Transparent | Transparent | Transparent | Transparent |
| Example 14 | Transparent | Transparent | Transparent | Transparent |
| Example 18 | Light blue | Light blue | Transparent | Transparent |
| Comparative Example 45 | Light brown | Purple | Dark purple | Dark purple |

By using the allergen reducing composition according to the present invention, an agent which can be sprayed to interior commodities such as a floor in a room, a carpet, a sofa, wall paper, and a curtain; bedding such as Futon ticking, a Futon cover, Futon padding, a sheet, a pillow cover, and a mat; automotive parts such as a car seat and a car mat; or stuffed toys can be provided. In addition, by processing unwoven fabric, textile, or a textile product, or an architectural interior material with the allergen reducing composition, a filter material, unwoven fabric such as a mask, textile, or a textile product, and an architectural interior material such as a floor material, a ceiling material, and wall paper, each of which has a function of reducing allergens such as mites and cedar pollen can be provided. In addition, problems related to coloring, color changing over time, and corrosion of iron, which cannot be avoided in the conventional technology using the tannic acid, can be solved.

### INDUSTRIAL APPLICABILITY

By using the allergen reducing composition according to the present invention, which contains a zinc salt of gluconic acid, citric acid, lactic acid or malic acid or copper gluconate and a rare earth salt, allergens such as mites and pollen can be reduced, and in addition, without coloring, an allergen reducing composition for imparting a function of reducing allergens to a textile structure such as unwoven fabric, textile, or a textile product; unwoven fabric, textile, or a textile product capable of reducing allergens; and architectural interior materials capable of reducing the allergens such as wall paper, a floor material, and a ceiling material can be provided.

## Claims

1. An allergen reducing composition comprising:
(A) one or more kinds of compounds selected from the group consisting of a zinc salt of gluconic acid, citric acid, lactic acid or malic acid and copper gluconate; and
(B) one or more kinds of compounds selected from the group consisting of rare earth salts.

2. The allergen reducing composition according to claim 1, wherein the zinc salt is zinc gluconate.

3. The allergen reducing composition according to claim 1 or claim 2, wherein the rare earth salt is one or more kinds of compounds selected from the group consisting of a lanthanum salt, a cerium salt, a gadolinium salt, an erbium salt, and an ytterbium salt.

4. The allergen reducing composition according to any one of claim 1 to claim 3, wherein the rare earth salt is a lanthanum salt or a cerium salt.

5. The allergen reducing composition according to any one of claim 1 to claim 4, wherein a mixing ratio of (A) and (B) is 1 : 9 to 9 : 1 in a weight ratio.

6. The allergen reducing composition according to any one of claim 1 to claim 5, including 0.5 to 70 wt.% (A) and (B) in total.

7. A spray agent comprising:
the allergen reducing composition according to any one of claim 1 to claim 6; and water.

8. A surface treatment agent comprising:
the allergen reducing composition according to any one of claim 1 to claim 6; and water.

9. A method comprising processing a textile structure or an architectural interior material by the allergen reducing composition according to any one of claim 1 to claim 6.

10. An allergen reducing method comprising causing the allergen reducing composition according to any one of claim 1 to claim 6 to be present in an allergen reduction target space, wherein a method of treatment of a human or animal body is excluded.

11. An allergen reducing method comprising spraying the spray agent according to claim 7 in an allergen reduction target space, wherein a method of treatment of a human or animal body is excluded.

12. An allergen reducing method comprising causing the allergen reducing composition according to any one of claim 1 to claim 6 to be present on a surface of an allergen reduction target, wherein a method of treatment of a human or animal body is excluded.

13. An allergen reducing method comprising causing the surface treatment agent according to claim 8 to be present on a surface of an allergen reduction target, wherein a method of treatment of a human or animal body is excluded.

## Patentansprüche

1. Allergenreduzierende Zusammensetzung, umfassend
(A) eine oder mehrere Arten von Verbindungen, ausgewählt unter einem Zinksalz von Gluconsäure, Zitronensäure, Milchsäure oder Äpfelsäure und Kupfergluconat; und
(B) eine oder mehrere Arten von Verbindungen, ausgewählt unter Seltenerdmetallsalzen.

2. Allergenreduzierende Zusammensetzung nach Anspruch 1, wobei das Zinksalz Zinkgluconat ist.

3. Allergenreduzierende Zusammensetzung nach Anspruch 1 oder Anspruch 2, wobei das Seltenerdmetallsalz eine oder mehrere Arten von Verbindungen ist, ausgewählt unter einem Lanthansalz, einem Cersalz, einem Gadoliniumsalz, einem Erbiumsalz und einem Ytterbiumsalz.

4. Allergenreduzierende Zusammensetzung nach einem der Ansprüche 1 bis 3, wobei das Seltenerdmetallsalz ein Lanthansalz oder ein Cersalz ist.

5. Allergenreduzierende Zusammensetzung nach einem der Ansprüche 1 bis 4, wobei das Mischungsverhältnis von (A) und (B) als Gewichtsverhältnis 1:9 bis 9:1 beträgt.

6. Allergenreduzierende Zusammensetzung nach einem der Ansprüche 1 bis 5, welche insgesamt 0,5 bis 70 Gew.-% (A) und (B) enthält.

7. Sprühmittel, umfassend:
die allergenreduzierende Zusammensetzung nach einem der Ansprüche 1 bis 6; und Wasser.

8. Oberflächenbehandlungsmittel, umfassend:
die allergenreduzierende Zusammensetzung nach einem der Ansprüche 1 bis 6; und Wasser.

9. Verfahren, bei dem man eine textile Struktur oder ein architektonisches Innenmaterial mit der allergenreduzierende Zusammensetzung nach einem der Ansprüche 1 bis 6 behandelt.

10. Allergenreduzierendes Verfahren, bei dem man bewirkt, dass die allergenreduzierende Zusammensetzung nach einem der Ansprüche 1 bis 6 an einem Zielort vorhanden ist, an dem ein Allergen reduziert werden soll, wobei ein Verfahren zur Behandlung eines menschlichen oder tierischen Körpers ausgeschlossen ist.

11. Allergenreduzierendes Verfahren, bei dem man das Sprühmittel nach Anspruch 7 an einen Zielort sprüht, an dem ein Allergen reduziert werden soll, wobei ein Verfahren zur Behandlung eines menschlichen oder tierischen Körpers ausgeschlossen ist.

12. Allergenreduzierendes Verfahren, bei dem man bewirkt, dass die allergenreduzierende Zusammensetzung nach einem der Ansprüche 1 bis 6 auf einer Oberfläche eines Zielortes vorhanden ist, an dem ein Allergen reduziert werden soll, wobei ein Verfahren zur Behandlung eines menschlichen oder tierischen Körpers ausgeschlossen ist.

13. Allergenreduzierendes Verfahren, bei dem man bewirkt, dass das Oberflächenbehandlungsmittel nach Anspruch 8 auf einer Oberfläche eines Zielortes vorhanden ist, an dem ein Allergen reduziert werden soll, wobei ein Verfahren zur Behandlung eines menschlichen oder tierischen Körpers ausgeschlossen ist.

## Revendications

1. Composition de réduction d'allergènes, comprenant :
(A) un ou plusieurs types de composés choisis dans le groupe constitué par un sel de zinc d'acide gluconique, d'acide citrique, l'acide lactique ou d'acide malique et le gluconate de cuivre ; et
(B) un ou plusieurs types de composés choisis dans le groupe constitué par les sels de terres rares.

2. Composition de réduction d'allergènes selon la revendication 1, dans laquelle le sel de zinc est le gluconate de zinc.

3. Composition de réduction d'allergènes selon la revendication 1 ou la revendication 2, dans laquelle le sel de terres rares est constitué d'un ou plusieurs types de composés choisis dans le groupe constitué par un sel de lanthane, un sel de cérium, un sel de gadolinium, un sel d'erbium, et un sel d'ytterbium.

4. Composition de réduction d'allergènes selon l'une quelconque parmi la revendication 1 à la revendication 3, dans laquelle le sel de terres rares est un sel de lanthane ou un sel de cérium.

5. Composition de réduction d'allergènes selon l'une quelconque parmi la revendication 1 à la revendication 4, dans laquelle un rapport de mélange de (A) et (B) va de 1:9 à 9:1 selon un rapport pondéral.

6. Composition de réduction d'allergènes selon l'une quelconque parmi la revendication 1 à la revendication 5, comportant de 0,5 à 70% en poids de (A) et (B) au total.

7. Agent de pulvérisation, comprenant
la composition de réduction d'allergènes selon l'une quelconque parmi la revendication 1 à la revendication 6 ; et
de l'eau.

8. Agent de traitement de surface, comprenant :
la composition de réduction d'allergènes selon l'une quelconque parmi la revendication 1 à la revendication 6 ; et
de l'eau.

9. Méthode comprenant le traitement d'une structure textile ou d'un matériau d'architecture d'intérieur par la composition de réduction d'allergènes selon l'une quelconque parmi la revendication 1 à la revendication 6.

10. Méthode de réduction d'allergènes, comprenant le fait de provoquer la présence de la composition de réduction d'allergènes selon l'une quelconque parmi la revendication 1 à la revendication 6, dans un espace cible de réduction d'allergènes, où une méthode de traitement d'un corps humain ou animal est exclue.

11. Méthode de réduction d'allergènes, comprenant la pulvérisation de l'agent de pulvérisation selon la revendication 7 dans un espace cible de réduction d'allergènes, où une méthode de traitement d'un corps humain ou animal est exclue.

12. Méthode de réduction d'allergènes, comprenant le fait de provoquer la présence de la composition de réduction d'allergènes selon l'une quelconque parmi la revendication 1 à la revendication 6, sur une surface d'une cible de réduction d'allergènes, où une méthode de traitement d'un corps humain ou animal est exclue.

13. Méthode de réduction d'allergènes, comprenant le fait de provoquer la présence de l'agent de traitement de surface selon la revendication 8, sur une surface d'une cible de réduction d'allergènes, où une méthode de traitement d'un corps humain ou animal est exclue.
